# EUROPÄISCHE PATENTANMELDUNG

(11) **EP 1 208 797 A2**
(43) Veröffentlichungstag der Anmeldung: **29.05.2002**
(21) Anmeldenummer: 01000661.7
(22) Anmeldetag: 26.11.2001
(51) Int. Cl.: A61B 6/03

(54) **Röntgendetektor mit integrierter Kühlung**

(30) Priorität: 27.11.2000 DE 10058818
(71) Anmelder: Philips Corporate Intellectual Property GmbH, 52064 Aachen (DE); Koninklijke Philips Electronics N.V., 5621 BA Eindhoven (NL)
(72) Erfinder: Busse, Falko, Philips Corp. I.P.GmbH, 52064, Aachen (DE); van der Broeck, Heinz, Philips Corp.I.P.GmbH, 52064, Aachen (DE)
(74) Vertreter: Volmer, Georg, Dipl.-Ing.

(57) **Zusammenfassung**

Die Erfindung betrifft ein Röntgenuntersuchungsgerät bei dem der Röntgendetektor 1 und die Röntgenstrahlungsquelle 2 mittels eines gemeinsamen Kühlkreislaufes einer Konstanthaltung der Temperatur und einer Kühlung unterzogen werden. Dazu wird dem Röntgendetektor 1 ein Kühlmedium mit konstanter Temperatur zugeführt, um diesen Röntgendetektor 1 unter gleichmäßigen Umgebungstemperaturen arbeiten zu lassen. Die somit erstmalig erhöhte Temperatur des Kühlmittels reicht immer noch aus, um eine Kühlung der Röntgenstrahlungsquelle 2 vorzunehmen. Deswegen wid das erwärmte Kühlmittel nach der Zuführung zum Röntgendetektor 1 der Röntgenstrahlungsquelle 2 zugeführt, wo ein zweiter Wärmeaustausch stattfindet, so dass gleichzeitig ohne einen zusätzlichen Kühlkreislauf die Röntgenstrahlungsquelle 2 gekühlt wird. Dies hat zum Vorteil, dass entsprechende Röntgenuntersuchungsgeräte mit einfachen Konstruktionen ausgeführt werden können und die in der Konstruktion verwendeten elektronischen Bauelemente durch die konstante niedrige Temperatur eine entsprechend erhöhte Lebensdauer aufweisen und das Gerät durch die Kühlung mit einer höheren mittleren Leistung betrieben werden kann.

## Beschreibung

Die Erfindung betrifft ein Röntgenuntersuchungsgerät mit einem Röntgendetektor und einer Röntgenstrahlungsquelle, die an einer Trägervorrichtung angeordnet sind Außerdem betrifft die Erfindung einen Röntgendetektor mit einer Sensoreinheit, die Röntgenstrahlung in elektrische Bildsignale wandelt und bei der an eine Sensoreinheit eine Verarbeitungseinheit angrenzt, die mehrere Verstärkereinheiten enthält. Desweiteren betrifft die Erfindung ein Verfahren zur Kühlung von Röntgenuntersuchungsgeräten.

In bildgebenden Röntgenuntersuchungsgeräten durchdringt die von einer Röntgenstrahlungsquelle ausgesandte Röntgenstrahlung einen zu untersuchenden Patienten oder Gegenstand und wird der unterschiedlichen Dichte und chemischen Zusammensetzung des zu untersuchenden Gewebes oder der Knochen entsprechend geschwächt. Die Röntgenstrahlung wird im Röntgendetektor in einem szintillierenden Material in Licht umgesetzt oder in direkt detektierbare Ladungsträger umgewandelt.

Röntgenuntersuchungsgeräte werden z. B. in der Chirurgie eingesetzt und bestehen bspw. aus einer beweglichen Konsole und einer daran befestigten beweglichen Trägervorrichtung, welche auf der einen Seite eine Röntgenstrahlungsquelle und auf der gegenüberliegenden Seite einen Bildaufnehmer oder den Röntgendetektor aufnimmt.

Hinsichtlich einer einfachen Mobilität wird das ganze System gewichtsmäßig ausgeglichen. Hierzu werden an bestimmten Stellen Ausgleichsgewichte angebracht. Die in die Röntgenstrahlungsquelle eingespeiste Leistung wird nahezu vollständig in Wärme umgewandelt, die hauptsächlich über das Gehäuse der Röntgenstrahlungsquelle abgegeben wird. Da die zulässige Außentemperatur des Röntgengerätes einen bestimmten Wert nicht überschreiten darf, muss die mittlere Leistung begrenzt werden und das Gehäuse muss eine ausreichend große Oberfläche aufweisen. Letzteres ist allerdings hinsichtlich des Gewichtes und des erforderlichen Platzbedarfes der bspw. für die Chirurgie benötigt wird, nur begrenzt realisierbar.

In der Zukunft werden die bisher verwendeten Bildaufnehmer oder Röntgendetektoren die aus einem Röntgenbildverstärker und einer Kamera bestehen durch flache dynamische Röntgendetektoren ersetzt. Dies wird beispielhaft in der EP 440282 beschrieben. Bei diesen Systemen wird eine erhebliche Gewichtseinsparung erreicht, die für die Mobilität enorm wichtig ist. Um diese Gewichtseinsparung aber für das Gesamtsystem wirksam werden zu lassen, muss auf der Seite der Röntgenstrahlungsquelle auch eine entsprechende Gewichtsreduzierung vorgenommen werden. Dies ist aber nur im eingeschränkten Maße möglich. Unter anderem verhindert die erforderliche Wärmeabgabe bei gleichbleibender Röntgenstrahlung eine ausreichend große Gewichtsreduzierung. Zusätzlich wird die Problematik dadurch vergrößert, dass mit einem flachen Röntgendetektor höhere mittlere Dauerleistungen möglich werden.

In der EP 0182040 wird eine Kühlvorrichtung für einen Computertomographen beschrieben. Bei Computertomographiegeräten entsteht durch die konstruktionsbedingte Anordnung eine sehr große Wärmemenge, die einerseits begrenzt werden muss, um den in dem Ring befindlichen zu untersuchenden Patienten nicht zu beeinflussen und andererseits die Lebensdauer des Computertomographiegerätes, speziell der Röntgenröhre zu verlängern Deshalb wird die Röntgenstrahlungsquelle mit einem Kühlmedium gekühlt. Aufgrund der Anordnung von Röntgenstrahlungsquelle und Röntgendetektor an einem Ring wird die Röntgenstrahlungsquelle durch einen im Ring befindliches Leitungssystem in dem sich Öl befindet, gekühlt. Das aufgrund der Wärmeabgabe der Röntgenstrahlungsquelle erhitzte Öl wird über eine Kühlaustrittsöffnung in den Ring befördert und dort von einer Umwälzpumpe um den Ring befördert, um über die Kühleintrittsöffnung wieder zur Röntgenstrahlungsquelle zu gelangen. Dabei wird der Weg des Ringes dazu benutzt, das erhitzte Kühlmedium abzukühlen. Die Abkühlung des Kühlöls wird außerdem noch durch im Ring befindliche luftenthaltende Kanäle ermöglicht. Dabei sind die Kühlkanäle in dem feststehenden Teils des Ringes angeordnet. Kühlrippen die am beweglichen Teil des Ringes angeordnet sind ragen in die Kühlkanäle in dem festen Teil des Ringes hinein und werden dort abgekühlt, so dass das im Leitungssystem befindliche Kühlöl abgekühlt werden kann. Hier wird eine direkte Kühlung der Röntgenstrahlungsquelle vorgeschlagen.

In der DE 29510802 U1 wird eine chirurgische Röntgendiagnostikeinrichtung beschrieben, bei der an einem C-Arm der Röntgengenerator und der Röntgenbildverstärker angeordnet sind Eine Kühlung des Röntgengenerators wird durch eine doppelwandige Ausführung des C-Arms ermöglicht.

Aufgabe der Erfindung ist es, eine Anordnung anzugeben, bei der eine Konstanthaltung der Temperatur im Röntgendetektor und gleichzeitig eine Niedrighaltung des Gesamtgewichts des gesamten Röntgenuntersuchungsgerätes ermöglicht wird.

Diese Aufgabe wird erfindungsgemäß dadurch gelöst, dass ein Röntgenuntersuchungsgerät vorgesehen ist, mit einer Trägervorrichtung einem Röntgendetektor und einer Röntgenstrahlungsquelle und einem Leitungssystem, das mit einem Wärmetauscher gekoppelt, dem Röntgendetektor und der Röntgenstrahlungsquelle zugeordnet und zur Aufnahme eines Kühlmediums vorgesehen ist.

Die Erfindung basiert auf dem Gedanken, dass ein Röntgendetektor sehr stabil arbeitet und gleichartige Bildsignale erzeugt, wenn alle seine Komponenten der gleichen Temperatur ausgesetzt sind. Davon ausgehend, dass die Temperatur in einem derartigen Röntgendetektor nicht sehr hoch ist, wird ein zur Konstanthaltung der Temperatur eingssetztes Kühlmedium nicht stark erwärmt. Die Röntgenstrahlungsquelle produziert wesentlich mehr Wärme als der Röntgendetektor. Deshalb wird das leicht erwärmte Kühlmedium, das in erster Linie zur Konstanthaltung der Temperatur im Röntgendetektor eingssetzt wird, zusätzlich zur Kühlung der Röntgenstrahlungsquelle eingssetzt. Dadurch erübrigt sich eine zusätzliche Kühlvorrichtung für die Röntgenstrahlungsquelle, wodurch die Komplexität eines Röntgenuntersuchungsgerätes und auch das Gesamtgewicht erheblich reduziert wird.

In einer vorteilhaften Ausgestaltung der Anordnung wird des Kühlmedium dem Röntgendetektor über ein erstes Teilleitungssystem mit konstanter Temperatur zugeführt.

Dadurch wird erreicht, das sowohl die Komponenten im Röntgendetektor unter gleichen Temperatur Bedingungen arbeiten, wodurch gleichmäßig verstärkte Bildsignale erzeugt werden. Außerdem wird die Röntgendetektorinnentemperatur niedrig gehalten, so dass die im Röntgendetektor befindliche Sensoreinheit zur Wandlung der Röntgenstrahlung in elektrische Signale im Bereich der höchsten Sensibilität arbeitet. Das Kühlmedium nimmt im Röntgendetektor eine erste Wärmemenge auf, die jedoch nicht sehr groß ist, so dass das Kühlmedium ohne weitere Kühlung der Röntgenstrahlungsquelle über ein zweites Teilleitungssystem zur weiteren Kühlung zugeführt wird. Nach der Kühlung der Röntgenstrahlungsquelle wird das erhitzte Kühlmedium einem Wärmetauscher zugeführt, in dem es auf die Ausgangstemperatur abgekühlt wird.

In einer vorteilhaften Ausgsstaltung der Erfindung sind im Röntgendetektor Temperatursensoren untergebracht, die ein temperaturabhängiges Signal zum Wärmeaustauscher zuführen, wodurch eine Temperaturregelung ermöglicht wird. Der Wärmetauscher ist vorzugsweise außerhalb der Trägervorrichtung anzuordnen, wobei jedoch bei entsprechend kleiner Ausführung des Gesamtsystems ein kompakter Wärmeaustauscher auch in den C-Arm integriert oder an dem C-Arm angsordnet werden kann.

Vom Wärmetauscher wird das Kühlmedium, vorzugsweise Wasser, zunächst mit niedriger aber konstanter Temperatur bspw. 20°C zum Detektor geführt, um dort die erforderliche Temperaturstabilisierung vorzunehmen. Die dann etwas erwärmte Kühlflüssigkeit z. B. auf 25°C, wird dann weiter zum Gehäuse der Röntgenstrahlungsquelle geführt, um dort weitere Wärme aufzunehmen. Die Röntgenstrahlungsquelle befindet sich in einem Gehäuse und besteht im wesentlichen aus der Röntgenröhre. Zusätzlich wird im gleichen Gehäuse optional auch der Hochspannungstransformator mit Gleichrichtung untergsbracht, wobei die Leistungszufuhr nicht bei Hochspannung erfolgen muss. In der Konsole befindet sich die Stromversorgung und die Steuerung. Nach der Kühlung der Röntgenstrahlungsquelle wird die Kühlflüssigkeit mit erhöhter Temperatur dem Wärmetauscher zugeführt, wo sie wieder auf eine niedrigere Temperatur abgekühlt wird. Die Flüssigkeitsleitung kann dabei über die Trägervorrichtung oder den C-Arm erfolgen. Der Wärmetauscher kann derart einfach ausgeführt sein, dass er nur die konstante niedrige Temperatur für den Detektor bereitstellt. Jede Wärmeentnahme im Gehäuse der Röntgenstrahlungsquelle reduziert die direkte Wärmeabgabe und erlaubt somit eine neue Konstruktion des Röntgenuntersuchungsgerätes mit geringerem Gewicht bzw. mit einer höheren mittleren Dauerleistung des Röntgenuntersuchungsgerät.

Mittels dieser Anordnung wird erreicht, dass die Röntgenstrahlungsquelle eine maximale Temperatur nicht überschreitet und gleichzeitig gewährleistet wird, dass der Röntgendetektor mit einer konstanten Temperatur betrieben wird.

Die Aufgabe der Erfindung wird auch durch einen Röntgendetektor mit einer Sensoreinheit gelöst, wobei die Sensoreinheit die Röntgenstrahlung in elektrische Signale wandelt und an die Sensoreinheit eine Verarbeitungseinheit angrenzt, die mehrere Verstärkereinheiten enthält. Die mehreren Verstärkereinheiten sind der Kühleinheit derart zugeordnet, dass sie eine Konstanthaltung der Temperatur aller Verstärkereinheiten ermöglichen bei gleichzeitiger Kühlung der in der Sensoreinheit befindlichen Szintillatoreinheit.

Dynamische Röntgendetektoren werden im Bereich der medizinischen Diagnostik eingesetzt. Sie gelten als universelle Detektorkomponenten, die in verschiedenen anwendungsspezifischen Röntgengeräten verwendet werden können. Eine wichtigs Eigenschaft dabei ist die Möglichkeit, Röntgenbilder und Röntgenbildsequenzen mit geringen Röntgenstrahlungsdosen aufnehmen zu können. Hierzu wird ein möglichst hohes Signal-zu-Rausch-Verhältnis des Röntgendetektors angestrebt. Um einen stabilen Betrieb der flachen dynamische Röntgendetektoren zu ermöglichen, ist es deshalb erforderlich die Temperatur in diesem Röntgendetektor konstant zu halten. Um dies mit entsprechender Effektivität und Zuverlässigkeit zu gewährleisten, wird erfindungsgemäß eine Fremdkühlung vorgeschlagen.

Flache dynamische Röntgendetektoren weisen einen schichtartigen Aufbau auf. Die Sensoreinheit enthält eine Szintillatoreinheit um die auftreffende Röntgenstrahlung in sichtbares Licht zu wandeln. Dieser Szintillatoreinheit ist eine lichtempfindliche Ausleseeinheit zugeordnet, die bspw. durch Fotodioden oder Fototransistoren realisiert sein kann. Die ausgelesenen Ladungsträger werden einer Verarbeitungseinheit zugeführt, in der eine Verstärkung der ausgelesenen elektrischen Signale vorgenommen wird. Die Sensoreinheit ist in viele Bildpunkte unterteilt, die matrixförmig angeordnet sind Dabei werden die erzeugten Ladungsträger Zeilen- oder Spaltenweise ausgelesen und mehreren entsprechenden Bildbereichen zugsordneten Verstärkereinheiten zugeführt. Um eine einheitliche Verstärkung unter gleichmäßigen Bedingungen zu erhalten, ist es notwendig, dass all diese Verstärkereinheiten in den selben Temperaturbereichen betrieben werden. Dazu sind diese Verstärkungseinheiten erfindungsgemäß an einer Kühleinheit angeordnet. Der Kühleinheit wird ein Kühlmedium zugsführt, welches eine Konstanthaltung der Temperatur aller Verstärkereinheiten ermöglicht. Dazu wird der Kühleinheit ein Kühlmedium mit konstanter niedriger Temperatur bspw. von einem oben genannten Wärmetauscher zugeführt. Dadurch werden alle im Röntgendetektor befindlichen Verstärkereinheiten auf eine einheitliche Temperatur gebracht, wodurch alle unter den selben Temperaturbedingen die Bildsignale verstärken.

Ein weiterer Vorteil dieser Anordnung ergibt sich dadurch, dass durch die Anordnung einer Kühleinheit in einem derartigen Röntgendetektor die Röntgendetektorinnentemperatur niedrig gehalten werden kann, so dass die Empfindlichkeit der Szintillatoreinheit entsprechend eingestellt werden kann. Die Szintillatoreinheit weist die Eigenschaft auf, dass bei steigender Temperatur die Empfindlichkeit, Röntgenstrahlung in elektrische Ladung zu wandeln, sinkt.

In einer weiteren erfindungsgemäßen Anordnung wird vorgeschlagen, einen Röntgendetektor mit einer erfindungsgemäßen Kühleinrichtung zu versehen, bei dem der Röntgendetektor eine Direktwandlung von Ladungsträgern in elektrische Signale vornimmt.

Ein weiterer Vorteil, der sich durch die erfindungsgemäße Anordnung ergibt, liegt darin, dass durch die Niedrighaltung der Temperatur sowohl im Röntgendetektor als auch speziell an den einzelnen Verstärkungseinheiten eine erhöhte Lebensdauer der zum Betrieb der erforderlichen Komponenten notwendigen elektronischen Bauelemente ermöglicht wird.

Die Aufgabe wird auch durch ein Verfahren zur Kühlung von Röntgenuntersuchungsgeräten gelöst, bei dem ein Kühlmedium einem Röntgendetektor zugeführt wird und nach einem ersten Wärmeaustausch zwischen Kühlmedium und Röntgendetektor das erwärmte Kühlmedium zur Kühlung der Röntgenstrahlungsquelle zugeführt wird.

Nachfolgend wird anhand der Zeichnungen ein Ausführungsbeispiel der Erfindung näher erläutert. Es zeigen:
- Fig. 1: Röntgenuntersuchungsgerät
- Fig. 2: Kühlkreislauf mit Röntgendetektor und Röntgenstrahlungsquelle
- Fig. 3: dynamischen Röntgendetektor im Detail

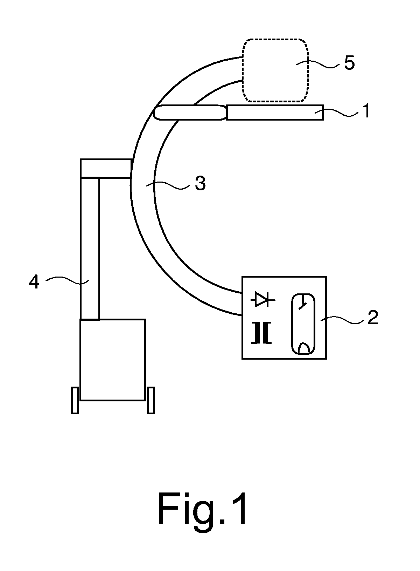

Fig.1 zeigt ein Röntgenuntersuchungsgerät, ausgeführt als C-Arm Röntgenuntersuchungsgerät. An der Trägervorrichtung 3, die an der Konsole 4 befestigt ist, ist sowohl der flache dynamische Röntgendetektor 1 und die Röntgenstrahlungsquelle 2 angeordnet. Die Trägervorrichtung 3 ist in diesem Ausführungsbeispiel als C-Arm realisiert. Der flache dynamische Röntgendetektor 1 kann auch, wie durch die Einheit 5 dargestellt, durch einen konventionellen Bildverstärker ersetzt werden.

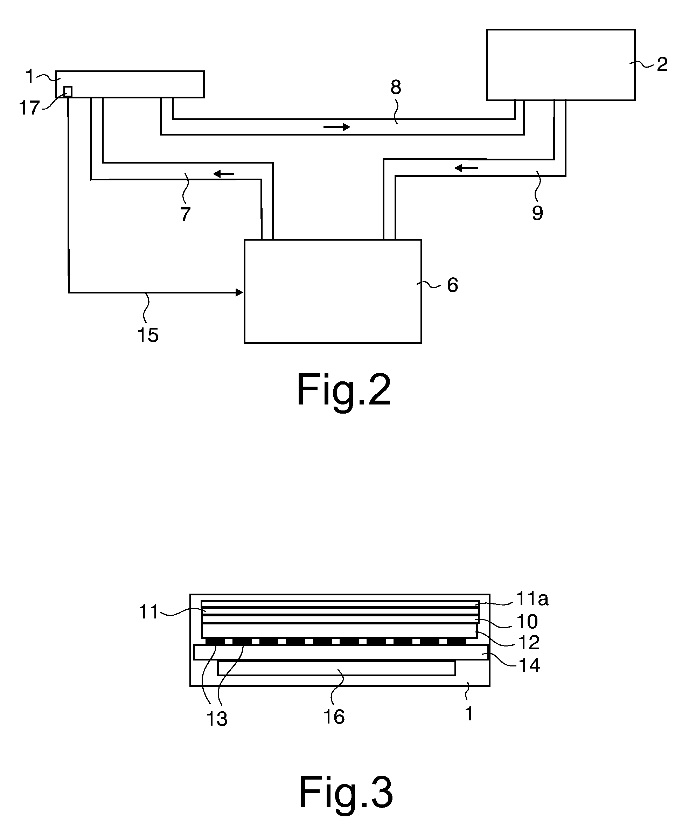

In Fig.2 ist der Kühlkreislauf zwischen dem Wärmetauscher 6, dem dynamischen Röntgendetektor 1 und der Röntgenstrahlungsquelle 2 dargestellt. Dabei wird das Kühlmedium in Form von Wasser oder Öl mit einer konstanten Temperatur von 20°C im ersten Teilleitungssystem 7 des Leitungssystems dem Röntgendetektor 1 zugeführt. Dort findet ein erster Wärmeaustausch zwischen dem Röntgendetektor 1 und dem Kühlmedium statt. Das Kühlmedium, das jetzt eine Temperatur von 25°C aufweist, wird über ein zweites Teilleitungssystem 8 der Röntgenstrahlungsquelle 2 zugeführt. Die Röntgenstrahlungsquelle produziert Temperaturen die über 50° C liegen. Durch die Zuführung des Kühlmediums zur Röntgenstrahlungsquelle wird ein zweiter Wärmeaustausch ermöglicht, der die Temperatur der Röntgenquelle auf eine maximale Höchsttemperatur von 50° C reduziert. Dieser zweite Wärmeaustausch erhitzt das Kühlmedium auf z.B. 35°C. Das nochmals erwärmte Kühlmedium wird nun im dritten Teilleitungssystem 9 dem Wärmetauscher 6 zugeführt, in dem das Kühlmedium wieder auf die Temperatur von 20°C abgekühlt wird. Im Röntgendetektor sind ein oder mehrere Temperatursensoren 17 angeordnet, um ein von der Temperatur im Röntgendetektor abhängiges Signal 15 erzeugen, welches dem Wärmetauscher 6 über die Leitung 15 zur Temperaturregelung zugeführt wird.

In Fig.3 ist ein dynamischer Röntgendetektor 1 im Detail dargestellt. Der Röntgendetektor 1 besteht aus einer Röntgenkonvertereinheit 11a, einer Sensorschicht 11 und einer Glasschicht 10, die unter der Sensoreinheit 11 angeordnet ist. Unter der Sensoreinheit 11 befindet sich eine Verarbeitungseinheit 12, die mehrere Verstärkereinheiten 13 umfasst. Diese Verstärkereinheiten 13, die zur Verstärkung der ausgelesenen Bildsignale einzelner Bildbereiche der Sensoreinheit 11 dienen, produzieren Wärme. Die Verstärkereinheiten 13 sind derart angeordnet, dass sie an die Kühleinheit 14 angrenzen und somit eine kontinuierliche Temperaturreduzierung erfolgen kann, so dass alle Verstärkereinheiten 13 auf dem selben niedrigen Temperaturniveau betrieben werden können. Dadurch wird es ermöglicht, dass alle Bildbereiche der Sensoreinheit 11 mit den selben Bedingungen verstärkt werden und so keine fehlerhaften Darstellungen des Gesamtbildes erzeugt werden. Der Kühleinheit 14 wird über die in Fig. 3 nicht dargestellte erste Teilleitung 7 das Kühlmedium mit einer konstanten Temperatur von 20°C zugeführt. Über eine ebenfalls nicht dargestellte zweite Teilleitung 8 wird das Kühlmedium von dem Röntgendetektor 1 wegbewegt. Eine weitere Verarbeitungseinheit 16 dient der Weiterverarbeitung der verstärkten Bildsignale und der Übertragung zu Peripheriegeräten.

## Patentansprüche

1. Röntgenuntersuchungsgerät mit einer Trägervorrichtung (3) einem Röntgendetektor (1) und einer Röntgenstrahlungsquelle (2) und
einem Leitungssystem (7, 8, 9), das mit einem Wärmetauscher (6) gekoppelt, dem Röntgendetektor (1) und der Röntgenstrahlungsquelle (2) zugeordnet und zur Aufnahme eines Kühlmediums vorgesehen ist.

2. Röntgenuntersuchungsgerät nach Anspruch 1,
**dadurch gekennzeichnet,**
**dass** vorgesehen ist, das Kühlmedium mittels eines ersten Teilleitungssystems (7) dem Röntgendetektor (1) zur Konstanthaltung der Temperatur und nach einem ersten Wärmeaustausch zwischen Kühlmedium und Röntgendetektor (1) über ein zweites Teilleitungssystem (8) der Röntgenstrahlungsquelle (2) und über ein drittes Teilleitungssystem (9) dem Wärmetauscher (6) zuzuführen.

3. Röntgenuntersuchungsgerät nach Anspruch 1,
**dadurch gekennzeichnet,**
**dass** der Röntgendetektor (1) Bildpunktsensoren enthält, die matrixförmig angeordnet sind und Gruppen von Bildpunktsensoren Verstärkereinheiten (13) zur Verstärkung von Bildsignalen zugeordnet sind

4. Röntgenuntersuchungsgerät nach Anspruch 1,
**dadurch gekennzeichnet,**
**dass** vorgesehen ist, dem Röntgendetektor das Kühlmedium mit konstanter Temperatur zuzuführen.

5. Röntgenuntersuchungsgerät nach Anspruch 1,
**dadurch gekennzeichnet,**
**dass** das Kühlmedium nach einem ersten Wärmeaustausch mit dem Röntgendetektor ohne Temperaturabsenkung oder Kühlung der Röntgenstrahlungsquelle zuführbar ist.

6. Röntgenuntersuchungsgerät nach Anspruch 1,
**dadurch gekennzeichnet,**
**dass** dem Röntgendetektor ein Temperatursensor (17) zur Erzeugung eines Temperatur abhängigen Signals (15) zugeordnet ist und vorgesehen ist, dass Temperatursignal (15) dem Wärmetauscher (6) zur Temperaturregelung des Kühlmediums zuzuführen.

7. Röntgenuntersuchungsgerät nach Anspruch 1,
**dadurch gekennzeichnet,**
**dass** der Wärmetauscher (6) außerhalb der Trägervorrichtung (3) angeordnet ist.

8. Röntgendetektor mit einer Sensoreinheit (11), die die Röntgenstrahlung in elektrische Signale wandelt, einer an die Sensoreinheit (11) grenzenden Verarbeitungseinheit (12) mit mehreren Verstärkereinheiten (13), die einer Kühleinheit (14) zugeordnet sind, die ein Kühlmedium enthält, welches eine Konstanthaltung der Temperatur aller Verstärkungseinheiten (13) und eine Kühlung der in der Sensoreinheit (11) befindlichen Röntgenkonvertereinheit (11a) ermöglicht.

9. Verfahren zur Kühlung von Röntgenuntersuchungsgeräten, bei dem ein Kühlmedium einem Röntgendetektor (1) zugeführt wird und nach einem ersten Wärmeaustausch zwischen Kühlmedium und Röntgendetektor (1) das erwärmte Kühlmedium zur Kühlung der Röntgenstrahlungsquelle (2) zugeführt wird.
